# EUROPÄISCHE PATENTSCHRIFT

(11) **EP 0 367 103 B1**
(45) Veröffentlichungstag und Bekanntmachung des Hinweises auf die Patenterteilung: **07.01.1993**
(21) Anmeldenummer: 89119890.5
(22) Anmeldetag: 26.10.1989
(51) Int. Cl.: A61K 31/575, A61K 47/00

(54) **Topische Salbe**
Topical ointment
Onguent topique

(30) Priorität: 31.10.1988 DE 3836971
(43) Veröffentlichungstag der Anmeldung: 09.05.1990
(73) Patentinhaber: Flender, Gabriele, D-97980 Bad Mergentheim (DE); Schürmann, Gabriele, D-97980 Bad Mergentheim (DE)
(72) Erfinder: Flender, Gabriele, D-6991 Igersheim (DE)
(74) Vertreter: Durm, Frank, Dipl.-Ing.

(56) Entgegenhaltungen:
- EP-A- 0 098 568
- DE-A- 3 526 669

## Beschreibung

Die Erfindung betrifft die Verwendung von Cholesterin zur Herstellung einer Arzneimittels für die topische Behandlung von Dermatosen, insbesondere von Psoriasis, die Bereitstellung einer topischen Salbe zur Behandlung von Dermatosen, insbesondere von Psoriasis (Shuppenflechte), sowie ein Verfahren zur Herstellung einer derartigen Salbe.

Es sind vielerlei Salben zur Behandlung dermatologischer Störungen bekannt, welche auf die erkrankten Hautstellen aufgetragen werden. Zur Behandlung von Psoriasis und Ekzemen werden vorzugsweise Salben verwendet, welche Corticosteroide oder Salicylsäure als pharmazeutisch wirksame Substanzen enthalten. Es hat sich jedoch immer wieder gezeigt, daß diese Salben oftmals nicht die gewünschte heilende Wirkung haben. Zudem kommt es bei Patienten, die mit cortisonhaltigen Externa behandelt wurden, zu gravierenden Nebenwirkungen, wie zum Beispiel einer nicht heilbaren Hautalterung (Atrophie). Ebenfalls häufig sind Hautstreifen (Striae), Infektionsverschlimmerungen und andere bleibende Hautschäden (Teleangiektasien). Weitere mögliche Nebenwirkungen herkömmlicher Salben und Cremes sind: sogenannte Steroid-Akne, fleckförmige bis flächenhafte Hautblutungen und Hautgeschwüre im Bereich des Auftrags.

Eine kortikoidhaltige Zubereitung zur topischen Applikation ist beispielweise aus der EP-A3-0 098 568 bekannt.

Die DE-OS 35 26 669 beschreibt ein Emulsionssystem des Wasser-in-Öl-Typs auf der Basis eines Erdalkali- oder metallischen Lanolates als Trägermaterial für einen in der Kosmetik oder Therapie aktiven Bestandteil, wobei die Zusammensetzung in Form einer Creme vorliegt.

Aus der DE-PS 638 839 ist ein Verfahren zur Herstellung einer wässerig-alkoholischen Lösung bekannt, welche Cholesterin enthält und für therapeutische Zwecke, insbesondere für die Behandlung der Kopfhaut, geeignet sein soll.

Die DE-OS 35 26 669 offenbart in Beispiel 6 eine feuchtigkeitsspendende Creme, die als Bestandteile Vaseline-Öl und Cholesterol aufweist. Ein solches Emulsionssystem könne zur Herstellung von kosmetischen und pharmazeutischen Emulsionen verwendet werden.

In der DE-OS 31 25 710 ist ein kosmetisches Mittel, unter anderem in Form von Öl-in-Wasser-Dispersionen, beschrieben. Beispiel 4 betrifft ein pflegendes Fluid, das unter anderem auch Cholesterin enthält.

Kosmetische Produkte, die in Öl gelöstes Cholesterin enthalten, sind ferner auch aus der FR-PS 9 280 337 bekannt.

In der DE-OS 37 13 492 wird ein Konzentrat für die Behandlung gereizter Haut vorgeschlagen, welches neben einem Lipid, Wasser und Glyzerin auch Cholesterin enthält.

In der DE-OS 33 19 304 ist ein Arzneimittel mit antineoplastischer Wirkung beschrieben, welches neben einem lipophilen Lösungsmittel auch Cholesterin enthält.

In der CH-PS 1 43 520 sind die kosmetischen Eigenschaften von Cholesterin, das in einer wässerigen bzw. wässerig-alkoholischen Lösung enthalten ist, beschrieben.

In "Pharmazeutische Technologie", herausgegeben von Sucker, Fuchs und Speiser, Stuttgart 1978, Seiten 305 - 310, wird Cholesterin als Emulgator in Dermatika erwähnt. Auf Seite 659 findet sich zudem der Hinweis, daß Wollfett auf der Haut verstreichbar ist und einen erweichenden Effekt auf die Hornschicht bewirkt.

Schließlich enthält Janistyn: "Handbuch der Kosmetika und Riechstoffe", I. Band, Heidelberg 1969, Seite 538, den Hinweis, daß Jojoba-Öl als Wundheilmittel und als Haaröl Verwendung findet.

Die Gruppe der Patienten, bei denen die eingangs geschilderten Nebenwirkungen auftreten, nimmt in jüngerer Zeit aufgrund umweltbedingten Belastungen rapide zu. Solche Patienten sind, auch infolge von allergischen Reaktionen auf die üblicherweise verwendeten Konservierungsstoffe, mit den bekannten pharmazeutischen Externa nicht mehr therapierbar.

Aufgabe der Erfindung war somit die Bereitstellung eines hochwirksamen und nebenwirkungsfreien Wirkstoffes sowie eines Arzneimittels zur topischen Behandlung von Dermatosen, insbesondere von Psoriasis. Ferner wird ein Verfahren zur Herstellung einer solchen Salbe angegeben.

Es hat sich gezeigt, daß Cholesterin die gewünschte pharmazeutische Wirkung hat.

Eine erfindungsgemäß zusammengesetzte Salbe besteht aus 10 bis 50 Gewichtsprozent Vaseline, 10 bis 50 Gewichtsprozent Jojobaöl, 1 bis 10 Gewichtsprozent Cholesterin, 1 bis 10 Gewichtsprozent Bienenwachs oder Walrat bzw. Walratersatz sowie 10 bis 40 Gewichtsprozent Wasser.

Vaseline ist ein weiches bis salbenartig festes Gemisch aus festen und flüssigen Erdölkohlenwasserstoffen mit einem Schmelzbereich zwischen 35 und 60 Grad Celsius. Handelsüblich sind gelbe Vaseline (Vaselinum flavum) und gebleichte weiße Vaseline (Vaselinum album). Die hier bevorzugte weiße, hochgereinigte Vaseline dient als inerte Trägersubstanz der Salbe.

Das aus der Jojoba-Pflanze gewonnene Öl dient ebenfalls, gemeinsam mit der Vaseline, als Trägersubstanz, entfaltet darüber hinaus jedoch auch heilende Wirkung.

Wesentliche pharmazeutisch wirksame Substanz ist jedoch das Cholesterin, das mit einem relativ hohen Gewichtsanteil in der Salbe enthalten ist. Cholesterin als pharmazeutischer Wirkstoff zur Behandlung von Dermatosen, insbesondere Psoriasis, ist aus dem vorstehend abgehandelten druckschriftlichen Stand der Technik nicht bekannt.

Das Cholesterin gehört chemisch zu den Steroiden, hat jedoch nicht die antiinflammatorische Aktivität der Nebennierenrindenhormone, die sich von den Steroiden ableiten. Damit sind auch nicht deren hinreichend bekannte Nebenwirkungen in Kauf zu nehmen. Das Cholesterin wird bevorzugt in hochreiner Form (Cholesterinum purissimum) verwendet. Zur Herstellung der Salbe kann aus Wollfett gewonnenes Cholesterin in Form von kristallisiertem Pulver verwendet werden.

Weißes oder gelbes Bienenwachs oder auch Walrat bzw. Walratersatz sowie der Anteil an Wasser (Aqua dest.) beeinflussen die Konsistenz als gut streichfähige Salbe. Walrat (Cetaceum) ist eine weiße, wachsartige Masse, die aus der Kopfhöhle des Pottwals gewonnen wird und chemisch vorwiegend aus Palmetinsäureacetylester besteht. Heute wird anstelle echten Walrats meist künstlich hergestellter Walratersatz gleicher chemischer Zusammensetzung verwendet.

Das in der Salbe erfindungsgemäßer Zusammensetzung enthaltene Cholesterin wirkt als Lipid emulgierend. Als körperähnliche Substanz führt der Auftrag auf erkrankte Hautflächen nicht zu Unverträglichkeiten oder allergischen Reaktionen. Die erfindungsgemäße Salbe stellt somit das ideale Ausweichpräparat zur Behandlung von Dermatosen für solche Patienten dar, die keine der herkömmlichen Präparate auf der Haut vertragen. Für die mit den herkömmlichen Präparaten nicht mehr therapierbaren Patienten stellt die vorgeschlagene Salbe eine neue Perspektive dar. Für eine wachsende Anzahl von Patienten ist die Salbe gemäß der Erfindung die einzige, bisher bekanntgewordene Möglichkeit, einen nebenwirkungsfreien Therapieerfolg zu erzielen.

Besonders wirksam für die Behandlung von Psoriasis hat sich. eine Salbe erwiesen, die aus 20 bis 40 Gewichtsprozent Vaseline, 25 bis 45 Gewichtsprozent Jojobaöl, 2 bis 6 Gewichtsprozent Cholesterin, 2 bis 8 Gewichtsprozent Bienenwachs, Walrat oder Walratersatz sowie 15 bis 30 Gewichtsprozent Wasser besteht. Insbesondere wird eine Zusammensetzung aus 25 bis 35 Gewichtsprozent Vaseline, 35 bis 42 Gewichtsprozent Jojobaöl, 3 bis 5 Gewichtsprozent Cholesterin, 4 bis 7 Gewichtsprozent Bienenwachs, Walrat oder Walratersatz und 20 bis 25 Gewichtsprozent Wasser bevorzugt.

Die Salbe wird hergestellt durch sorgfältiges Mischen der Bestandteile bei erhöhter Temperatur. Dabei dienen Vaseline, Jojobaöl und Bienenwachs als Salbengrundlage, der das Cholesterin als pharmazeutisch wirksame Substanz einverleibt wird.

Vorzugsweise wird Vaseline, Jojobaöl, Cholesterin und Bienenwachs gemeinsam auf etwa 75 Grad Celsius erhitzt. Unter sorgfältigem Rühren entsteht ein dünnflüssiges zweiphasiges Gemisch. Je nach gewünschter Konsistenz wird eine entsprechende Menge destillierten Wassers hinzugegeben. Die anschließende Abkühlung auf Umgebungstemperatur sollte unter ständigem Weiterrühren erfolgen. Für dieses Herstellungsverfahren geeignete Geräte sind bekannt und umfassen Wärmetauscher und Mischer.

Der Salbe der vorgeschlagenen Zusammensetzung müssen keine Konservierungsstoffe zugesetzt werden. Eine ausreichende Haltbarkeit kann beispielsweise schon durch Vakuumverpackung erzielt werden. Für eine längere Haltbarkeit können jedoch auch übliche Konservierungsstoffe zugesetzt werden.

Die nachstehenden Beispiele erläutern die erfindungsgemäße Zusammensetzung der Salbe:

### Beispiel 1

- Vaseline: 32 Gewichtsprozent
- Jojobaöl: 38 Gewichtsprozent
- Cholesterin: 3 Gewichtsprozent
- Gelbes Bienenwachs: 5 Gewichtsprozent
- Aqua dest.: 22 Gewichtsprozent

### Beispiel 2

- Vaseline: 30 Gewichtsprozent
- Jojobaöl: 40 Gewichtsprozent
- Cholesterin: 4 Gewichtsprozent
- Walratersatz: 6 Gewichtsprozent
- Aqua dest.: 20 Gewichtsprozent

Die Salbe gemäß Beispiel 1 wurde in unter ärztlicher Leitung durchgeführten Untersuchungen an Patienten, welche unter chronischer therapieresistenter Psoriasis leiden, erprobt.

Bei einer ersten klinischen Untersuchung wurden insgesamt 27 Patienten therapiert. Dabei wurde jeweils die eine Körperhälfte des Patienten an den befallenen Hautstellen mit Salbe der in Beispiel 1 angegebenen Zusammensetzung behandelt, während die andere Körperhälfte mit einer salicylhaltigen Salbe behendelt wurde. Der Salbenauftrag erfolgte zweimal täglich. Der Behandlungsverlaut wurde jeweils nach einer, zwei und drei Wochen vom Untersuchungsleiter kontrolliert. Von den 27 behandelten Patienten waren 24 bereits nach ein oder zwei Wochen beschwerdefrei. Bei den übrigen drei Patienten zeigte sich zwar eine Wirkung, es kam jedoch nicht zur vollständigen Abheilung der Hautveränderungen.

An einer zweiten Untersuchung nahmen insgesamt 21 Patienten teil. Davon wurden 12 Patienten mit einer topischen Salbe gemäß Beispiel 1 behandelt, während die übrigen 9 Patienten mit einer topischen Salbe ohne Cholesterinzusatz, ansonsten jedoch gleicher Zusammensetzung, therapiert wurden. Die 9 behandelten Psoriasis-Patienten zeigten am Ende des 3-wöchigen Untersuchungszeitraumes keinerlei Änderungen ihres Hautstatus. Bei den anderen, mit der erfindungsgemäßen Salbe behandelten 12 Patientent, verschwanden die Psoriasis-Effloreszensen in 10 Fällen.

Während es bei vielen Patienten, die über einen längeren Zeitraum mit steroidhaltigen Salben therapiert werden, zu gravierenden Nebenwirkungen wie Atrophie, Teleangiektasien, Hautverdünnung und Striae im Bereich der betroffenen Hautstellen kommt, konnten derartige negative Begleiterscheinungen bei den mit der erfindungsgemäßen Salbe behandelten Patienten nicht festgestellt werden. Entsprechend positiv fielen die bei den durchgeführten Untersuchungen festgehaltenen Äußerungen der Patienten über das subjektive Empfinden aus.

## Patentansprüche (Patentansprüche für folgende(n) Vertragsstaat(en): AT, BE, CH, DE, FR, GB, IT, LI, LU, NL, SE)

1. Verwendung von Cholesterin zur Herstellung eines Arzneimittels für die topische Behandlung von Dermatosen, insbesondere von Psoriasis.

2. Topische Salbe zur Behandlung von Dermatosen, insbesondere Psoriasis, auf der Basis von Vaseline, Jojobaöl und Bienenwachs als Salbengrundlage, dadurch **gekennzeichnet**, daß sie Cholesterin als pharmazeutischen Wirkstoff enthält und sich zusammensetzt aus:
a) 10 bis 50 Gewichtsprozent Vaseline;
b) 10 bis 50 Gewichtsprozent Jojobaöl;
c) 1 bis 10 Gewichtsprozent Cholesterin;
d) 1 bis 10 Gewichtsprozent Bienenwachs, Walrat oder Walratersatz;
e) 10 bis 40 Gewichtsprozent Wasser.

3. Topische Salbe nach Anspruch 2 dadurch **gekennzeichnet**, daß sie zusammengesetzt ist aus:
a) 20 bis 40 Gewichtsprozent Vaseline;
b) 25 bis 45 Gewichtsprozent Jojobaöl;
c) 2 bis 6 Gewichtsprozent Cholesterin;
d) 2 bis 8 Gewichtsprozent Bienenwachs, Walrat oder Walratersatz;
e) 15 bis 30 Gewichtsprozent Wasser.

4. Topische Salbe nach Anspruch 3, dadurch **gekennzeichnet,** daß sie zusammengesetzt ist aus:
a) 25 bis 35 Gewichtsprozent Vaseline;
b) 35 bis 42 Gewichtsprozent Jojobaöl;
c) 3 bis 5 Gewichtsprozent Cholesterin;
d) 4 bis 7 Gewichtsprozent Bienenwachs, Walrat oder Walratersatz;
e) 20 bis 25 Gewichtsprozent Wasser.

5. Verfahren zur Herstellung der topischen Salben nach einem der Ansprüche 2 bis 4, dadurch **gekennzeichnet**, daß zunächst Vaseline, Jojobaöl, Cholesterin und Bienenwachs gemeinsam unter Rühren auf ungefähr 75 Grad Celsius erhitzt werden und anschließend dem so entstandenen dünnflüssigen Gemisch destilliertes Wasser zugesetzt wird.

6. Verfahren nach Anspruch 5, dadurch **gekennzeichnet**, daß die anschließende Abkühlung des Gemischs auf Umgebungstemperatur unter ständigem Weiterrühren erfolgt.

## Patentansprüche (Patentansprüche für folgende(n) Vertragsstaat(en): ES, GR)

1. Verfahren zur Herstellung einer topischen Salbe zur Behandlung von Dermatosen, insbesondere Psoriasis, unter Verwendung von Vaseline, Jojobaöl und Bienenwachs als Salbengrundlage, dadurch **gekennzeichnet,** daß Cholesterin als pharmazeutischer Wirkstoff einem Gemisch der nachfolgenden Zusammensetzung einverleibt wird:
a) 10 bis 50 Gewichtsprozent Vaseline;
b) 10 bis 50 Gewichtsprozent Jojobaöl;
c) 1 bis 10 Gewichtsprozent Cholesterin;
d) 1 bis 10 Gewichtsprozent Bienenwachs, Walrat oder Walratersatz
e) 10 bis 40 Gewichtsprozent Wasser.

2. Verfahren nach Anspruch 1, dadurch **gekennzeichnet**, daß folgende Anteile miteinander vermischt werden:
a) 20 bis 40 Gewichtsprozent Vaseline;
b) 25 bis 45 Gewichtsprozent Jojobaöl;
c) 2 bis 6 Gewichtsprozent Cholesterin;
d) 2 bis 8 Gewichtsprozent Bienenwachs, Walrat oder Walratersatz
e) 15 bis 30 Gewichtsprozent Wasser.

3. Verfahren nach Anspruch 2, dadurch **gekennzeichnet**, daß folgende Anteile miteinander vermischt werden:
a) 25 bis 35 Gewichtsprozent Vaseline;
b) 35 bis 42 Gewichtsprozent Jojobaöl;
c) 3 bis 5 Gewichtsprozent Cholesterin;
d) 4 bis 7 Gewichtsprozent Bienenwachs, Walrat oder Walratersatz;
e) 20 bis 25 Gewichtsprozent Wasser.

4. Verfahren nach einem der Ansprüche 1 bis 3, dadurch **gekennzeichnet**, daß zunächst Vaseline, Jojobaöl, Cholesterin und Bienenwachs gemeinsam unter Rühren auf ungefähr 75 Grad Celsius erhitzt werden und anschließend dem so entstandenen dünnflüssigen Gemisch destilliertes Wasser zugesetzt wird.

5. Verfahren nach Anspruch 4, dadurch **gekennzeichnet**, daß die anschließende Abkühlung des Gemischs auf Umgebungstemperatur unter ständigem Weiterrühren erfolgt.

## Claims (Claims for the following Contracting State(s): AT, BE, CH, DE, FR, GB, IT, LI, LU, NL, SE)

1. Use of cholesterol in the preparation of a medicament for the topical treatment of dermatoses, in particular psoriasis.

2. Topical ointment for treating dermatoses, in particular psoriasis, based on vaseline, jojoba oil and bees wax as ointment base, characterized in that it contains cholesterol as pharmaceutically active constituent and consists of:
a) 10 to 50% by wt. vaseline;
b) 10 to 50% by wt. jojoba oil;
c) 1 to 10% cholesterol;
c) 1 to 10% by wt. bees wax, spermaceti oil or spermaceti oil substitute;
e) 10 to 40% by wt. water.

3. Topical ointment according to Claim 2, characterized in that it consists of:
a) 20 to 40% by wt. vaseline;
b) 25 to 45% by wt. jojoba oil;
c) 2 to 6% by wt. cholesterol;
d) 2 to 8% by wt. bees wax, spermaceti oil or spermaceti oil substitute;
e) 15 to 30% by wt. water.

4. Topical ointment according to Claim 3, characterized in that it consists of:
a) 25 to 35% by wt. vaseline;
b) 35 to 42% by wt. jojoba oil;
c) 3 to 5% by wt. cholesterol;
d) 47 to 7% by wt. bees wax, spermaceti oil or spermaceti oil substitute;
e) 20 to 25% by wt. water.

5. Process for preparing the topical ointments according to one of Claims 2 to 4, characterized in that first of all vaseline, jojoba oil, cholesterol and bees wax are heated together while stirring to approximately 75°C and distilled water is then added to the resultant fluid mixture.

6. Process according to Claim 5, characterized in that the subsequent cooling of the mixture to ambient temperature takes place under continuous further stirring.

## Claims (Claims for the following Contracting State(s): ES, GR)

1. Process for preparing a topical ointment for treating dermatoses, in particular psoriasis, using vaseline, jojoba oil and bees wax as ointment base, characterized in that cholesterol is incorporated as pharmaceutically active substance in a mixture of the following composition:
a) 10 to 50% by wt. vaseline
b) 10 to 50% by wt. jojoba oil;
c) 1 to 10% by wt. cholesterol;
d) 1 to 10% by wt. bees wax, spermaceti oil or spermaceti oil substitute;
e) 10 to 40% by wt. water

2. Process according to Claim 1, characterized in that the following constituents are mixed with one another:
a) 20 to 40% by wt. vaseline;
b) 25 to 45% by wt. jojoba oil;
c) 2 to 6% by wt. cholesterol;
d) 2 to 8% by wt. bees wax, spermaceti oil or spermaceti oil substitute;
e) 15 to 30% by wt. water.

3. Process according to Claim 2, characterized in that the following constituents are mixed with one another :
a) 25 to 35% by wt. vaseline;
b) 35 to 42% by wt. jojoba oil;
c) 3 to 5% by wt. cholesterol;
d) 4 to 7% by wt. bees wax, spermaceti oil or spermaceti oil substitute;
e) 20 to 25% by wt. water.

4. Process according to one of Claims 1 to 3, characterized in that first of all vaseline, jojoba oil, cholesterol and bees wax are heated together while stirring to approximately 75°C and distilled water is then added to the resultant fluid mixture.

5. Process according to Claim 4, characterized in that the subsequent cooling mixture to ambient temperature takes place under constant further stirring.

## Revendications (Revendications pour l'(les) Etat(s) contractant(s) suivant(s): AT, BE, CH, DE, FR, GB, IT, LI, LU, NL, SE)

1. Mise en oeuvre de cholestérine pour la préparation d'un médicament pour le traitement de dermatoses, en particulier de psoriasis.

2. Pommade topique pour le traitement de dermatoses, en particulier de psoriasis, sur la base de vaseline, d'huile de jojoba et de cire d'abeilles comme base de la pommade, **caractérisée en ce** qu'elle contient de la cholestérine comme agent pharmaceutique actif et qu'elle est composée de :
a) 10 à 50 % en poids de vaseline,
b) 10 à 50 % en poids d'huile de jojoba,
c) 1 à 10 % en poids de cholestérine,
d) 1 à 10 % en poids de cire d'abeilles, de cétine ou de cétine de substitution
e) 10 à 40 % en poids d'eau.

3. Pommade topique selon la revendication 2, caractérisée en ce qu'elle est composée de :
a) 20 à 40 % en poids de vaseline,
b) 25 à 45 % en poids d'huile de jojoba,
c) 2 à 6 % en poids de cholestérine,
d) 2 à 8 % en poids de cire d'abeilles, de cétine ou de cétine de substitution,
e) 15 à 30 % en poids d'eau.

4. Pommade topique selon la revendication 3, caractérisée en ce qu'elle est composée de :
a) 25 à 35 % en poids de vaseline,
b) 35 à 42 % en poids d'huile de jojoba,
c) 3 à 5 % en poids de cholestérine,
d) 4 à 7 % en poids de cire d'abeilles, de cétine ou de cétine de substitution,
e) 20 à 25 % en poids d'eau.

5. Procédé de fabrication de la pommade topique selon l'une des revendications 2 à 4, caractérisé en ce que la vaseline, l'huile de jojoba, la cholestérine et la cire d'abeilles sont chauffées ensemble, en agitant, à environ 75°C et que de l'eau distillée est ajoutée ensuite au mélange très fluide ainsi obtenu.

6. Procédé selon la revendication 5, caractérisé en ce que le refroidissement consécutif du mélange à la température ambiante se fait en maintenant l'agitation.

## Revendications (Revendications pour l'(les) Etat(s) contractant(s) suivant(s): ES, GR)

1. Procédé de fabrication d'une pommade topique pour le traitement de dermatoses, en particulier de psoriasis, avec utilisation de vaseline, d'huile de jojoba et de cire d'abeilles comme base de la pommade, **caractérisé en ce** que la cholestérine est incorporée comme agent pharmaceutique actif dans un mélange présentant la composition suivante :
a) 10 à 50 % en poids de vaseline,
b) 10 à 50 % en poids d'huile de jojoba,
c) 1 à 10 % en poids de cholestérine,
d) 1 à 10 % en poids de cire d'abeilles, de cétine ou de cétine de substitution
e) 10 à 40 % en poids d'eau.

2. Procédé selon la revendication 1, caractérisé en ce que les constituants suivants sont mélangés les uns avec les autres :
a) 20 à 40 % en poids de vaseline,
b) 25 à 45 % en poids d'huile de jojoba,
c) 2 à 6 % en poids de cholestérine,
d) 2 à 8 % en poids de cire d'abeilles, de cétine ou de cétine de substitution,
e) 15 à 30 % en poids d'eau.

3. Procédé selon la revendication 2, caractérisé en ce que les constituants suivants sont mélangés les uns avec les autres :
a) 25 à 35 % en poids de vaseline,
b) 35 à 42 % en poids d'huile de jojoba,
c) 3 à 5 % en poids de cholestérine,
d) 4 à 7 % en poids de cire d'abeilles, de cétine ou de cétine de substitution,
e) 20 à 25 % en poids d'eau.

4. Procédé selon l'une des revendications 1 à 3, caractérisé en ce que la vaseline, l'huile de jojoba, la cholestérine et la cire d'abeilles sont chauffées ensemble, en agitant, à environ 75°C, et que l'eau distillée est ajoutée ensuite au mélange très fluide ainsi obtenu.

5. Procédé selon la revendication 4, caractérisé en ce que le refroidissement consécutif du mélange à la température ambiante se fait en maintenant l'agitation.
